# EUROPEAN PATENT APPLICATION

(11) **EP 3 028 679 A1**
(43) Date of publication of application: **08.06.2016**
(21) Application number: 14004092.4
(22) Date of filing: 04.12.2014
(51) Int. Cl.: A61F 2/95

(54) **Stent removal device**

(71) Applicant: Rheinisch-Westfälische Technische Hochschule Aachen, 52062 Aachen (DE)
(72) Inventor: Vazquez-Jimenez, Jaime, 52072 Aachen (DE); Honné, Axel, 52223 Stolberg (DE)
(74) Representative: Cohausz Hannig Borkowski Wißgott

(57) **Abstract**

The invention relates to a stent removal device comprising a tube (15) for feeding wires through the tube (15), the tube (15) having a distal end (15a) with a funnel shaped opening (16) for at least partially receiving a stent and a proximal end (15b), wherein the proximal end (15b) is connected to a supporting element (14, the supporting element (14) comprising a drive mechanism (17, 18, 19, 20) for fastening, particularly clamping and simultaneously pulling a plurality of wires fed through the tube (15). The invention furthermore relates to a sleeve (1) comprising an axial through hole, particularly a bore and a funnel shaped opening (3) of the through hole on one of its ends for facilitating feeding a plurality of wires (2) through the sleeve (1), and a crimping device for crimping such a sleeve (1).

## Description

The invention relates to a stent removal device comprising a tube for feeding wires through the tube, the tube having a distal end with a funnel shaped opening for at least partially receiving a stent and a proximal end. The invention furthermore relates to a sleeve for using in connection with the stent removal device, a crimping device for crimping the sleeve and a set comprising these elements.

Stent removal devices having the aforementioned features are known in the art. For example EP 1 827 304 B1 discloses such a device that may be used for repositioning a stent directly after inserting.

Stents are used for keeping a body lumen open. In typical applications stents are used in vessels or other passages of the human body, particularly in coronary vessels.

Sometimes stents that are already inplanted for longer tiome need to be removed from a body lumen. In one possible example this can occur after implanting a stent into a vessel of a growing child. Removal of such stent can be very problematic in view of the fact that newly formed tissue may have grown into the mesh structure of typical stents.

The aforementioned state of the art may be used for repositioning a stent by using the mentioned device in connection with a first wire fed through the mentioned tube, the first wire having a grip element at its distal end for engaging the positioned stent and for applying a pushing force to the stent and a second wire fed through the mentioned tube, the wire having a hook element at its distal end for engaging the implanted stent and for applying a pulling force to the stent. Using these two opposite forces it shall be possible to constrict the diameter of the proximal end of the positioned stent and to pull it into the funnel shaped tube, at least partially for repositioning.

It is a practical problem with this device that the entire pulling force is exerted to the stent by just using a single wire and the hook on its end. It may happen that the wire or the stent gets ruptured or the hook gets disengaged from the stent by bending. In addition the pulling force is exerted only to a single location of the stent thereby preventing a uniform constriction of the stent when pulling it. Particularly this device is not suitable to remove stents after a longer time.

It is an object of the invention to provide a device for removing a stent, even after longer times and particularly in an open surgery that provides a uniform and controllable pulling force to the stent without the risk of rupture of the pulling wire. Furthermore it is an object to provide additional devices to facilitate the usage of the stent removal device.

According to the invention this object is solved with a stent removal device having the aforementioned features in which furthermore the proximal end of the tube is connected to a supporting element, the supporting element comprising a drive mechanism for fastening, particularly clamping and simultaneously pulling a plurality of wires fed through the tube.

It is an essential feature of the invention to use a plurality of wires instead of a single one for exerting a pulling force to the proximal end of a stent that is to be removed. According to the invention it is intended to connect several wires, at least 3, along the periphery/circumference of the proximal end of the stent. For this purpose each wire may be fed through a mesh of the stent at its distal end like a sewing thread.

Preferably the locations where all the wires are connected to the stent are equally spaced along the circumference / periphery of the distal end. Both ends of each wire are used for pulling, consequently the stent is engaged by a closed loop of each wire in contrast to an open hook that may bend. In addition the totally exerted force is transferred to the stent by means of several wires instead of one thus reducing the risk of rupture of a single wire. Due to the fact that the wires are attached to the stent along its circumference at the distal end the total force is evenly distributed and the stent will constrict evenly when pulled, thereby preventing tissue damage. Due to the even distribution of the total force the total force may also be higher compared to the state of the art. Also stents having grown in tissue may be removed with such a device.

In order to accomplish that each wire will exert a part, particularly an equal part of the total force the removal device provides a driving mechanism that is used for fastening the plurality of wires after they are fed through the tube into the support element and that is used for simultaneously pulling all the wires together.

According to a first embodiment of the invention the drive mechanism of the removal may comprise a rotatable axis, particularly manually rotatable axis, the axis being supported by the supporting element and perpendicular to the direction of the extension of the plurality of wires in the proximal end of the tube and being capable to fasten the ends of the plurality of wires and to pull the plurality of wires through the tube by winding up their ends on the axis. The axis of rotation may intersect the direction of the plurality of wires fed through the tube in the proximal end of the tube.

Rotating the axis for example manually by a surgeon reduces the length of the wires beyond the distal end of the tube thus pulling the proximal end of the stent onto the funnel shaped opening of the tube, particularly when simultaneously moving the funnel shaped opening of the tube towards the stent, which may be performed by a surgeon if he moves the support element toward the stent.

According to another preferred embodiment the drive mechanism may comprise a rotatable axis, particularly manually rotatable axis, the axis being supported by the supporting element and parallel to the direction of the extension of the plurality of wires in the proximal end of the tube and a sliding element, the sliding element being engaged with a threaded part of the axis and being slidable within the support in a direction parallel to the axis by rotating the axis, the sliding element furthermore comprising a clamping element for fastening the respective end parts of the plurality of wires fed through the tube and fed at least into the sliding element, preferably through the sliding element.

In this embodiment a rotation of the axis will lead to an axial / linear movement of the sliding element to which all the wires of the aforementioned plurality of wires are fastened, particularly by means of clamping. The linear movement of the sliding element is parallel to the rotated axis and the fixed / clamped wires are moved preferably collinear to the extension in the proximal end part of the tube.

Both embodiments provide that all the wires are pulled simultaneously.

All the embodiment may be improved by a construction according to which the rotatable axis is connected to a manually operable knob, the knob protruding from the support. Accordingly it is easy for a surgeon to grip the knob and to turn it for pulling with one hand, particularly while gripping the support element with the other hand. Consequently it is an advantage if the supporting element forms a grip for manually gripping with a hand.

A clamping element for fastening the respective end parts of the plurality of wires may be formed by means of a screw screwed into a threaded bore hole of the sliding element, the axis of the screw crossing the axis of the wires fed into or through the sliding element. By tightly screwing the screw the plurality of wires is securely clamped and may be moved by operating the rotation axis.

In a preferred construction the rotatable axis and the middle axis of the proximal end of the tube that is fastened in the support element are parallel and offset. In addition this provides the possibility that the part of the tube emerging from the support towards a stent is bent in a plane, particularly in a plane comprising both mentioned axes. By using a bent tube it is more easy to access the field of surgery with the distal end of the tube and maintaining an unobstructed field of view. According to the invention it is intended to fed the plurality of open ends of the several wires through the tube and into a fastening or clamping part of the drive mechanism. In order to facilitate that the invention preferably uses a hollow sleeve, particularly made of aluminum or other metal into which the open ends of all the wires are fed prior to feeding them into and through the tube and drive mechanism.

A sleeve according to the invention may comprise an axial through hole, particularly bore and a funnel shaped opening of the through hole on at least one of its ends for facilitating feeding a plurality of wires through the sleeve. The sleeve furthermore is crimpable with a crimping device for fastening the sleeve on the plurality of wires. Attaching and fastening such a sleeve on the open ends of all wires keeps them together and helps to find the correct length of all wires prior to fastening the wires in the removal device. The sleeve now facilitates feeding the connected plurality of wires through the tube and or the sliding element of a stent removal device. Preferably the sleeve is not only fed into the sliding element but through the sliding element or another element for fastening the wires. That means that the in the sliding element still the wires are fastened, particularly by clamping and not the sleeve.

Handling of the sleeve may be improved if the external diameter of the sleeve is tapered, particularly in the middle of the axial extension since such shape may help to position the sleeve in a clamping device having a correspondingly formed recess for receiving a sleeve prior to crimping.

For the purpose of crimping the sleeve on the plurality of wires a crimping device may be used that comprises an upper cheek and a lower cheek (when put on a horizontal surface), both cheeks being movable with respect to each other for opening a respective recess in each of the opposing surfaces of the respective cheeks, particularly by means of a hinge connecting both cheeks, the recesses being adapted to receive a sleeve as previously described. In the closed state of the cheeks, when the opposing surface are in contact a channel exist, that exists in the interfacing area of the contacted surfaces and that is crossing the recesses and is collinear with the through hole of an inserted sleeve. Preferably a part of the channel may forming a funnel merging into the funnel of an inserted sleeve. Each recess may have a shape corresponding to a tapered sleeve, that helps to center the sleeve in the recess of the lower cheek.

The crimping device has a bolt in one of the cheeks, particularly the upper cheek, that is movable in a direction perpendicular to the channel and crossing at least one of the recesses, particularly the one in the upper cheek, and being adapted to crimp a sleeve inserted in the recesses with its tip. Furthermore a driving mechanism is provided particularly a manually operable lever, for driving the bolt and thus crimping the sleeve to the wires.

According to the invention it is intended to first put the sleeve into the crimping device, to close the cheeks and to fed the plurality of wires through the mentioned channel and the sleeve, simultaneously adjusting the required length of the wires. Afterwards the sleeve is crimped and connects all the wires that may now be fed into the removal device.

A preferred embodiment of the invention is shown in the figures and will be described in detail.

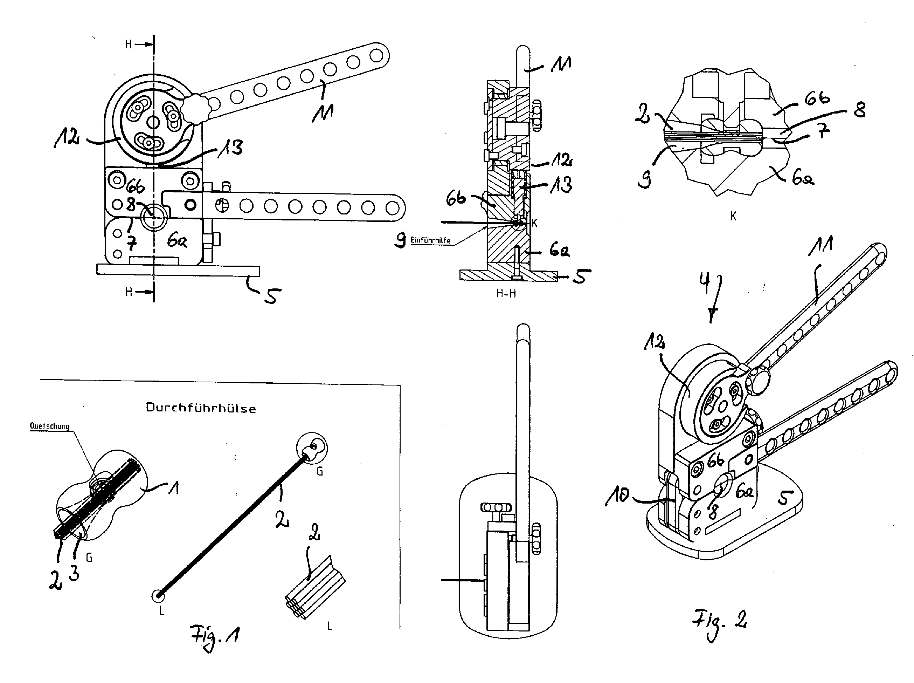

Figure 1 shows a sleeve 1 in accordance with the invention. The sleeve 1 has a longitudinal extension and is tapered in diameter at the middle of its length. The sleeve furthermore comprises a through hole or bore for feeding a plurality of wires 2 through the sleeve 1. In order to facilitate the insertion of the wires at least one axial end of the sleeve has a funnel shaped opening 3. For crimping the sleeve 1 and thus fixing the wires 2 in the sleeve a crimping device is used that is shown in figure 2.

This crimping device 4 comprises a base plate 5, a lower cheek 6a and an upper cheek 6b. In the interface area of the two opposing surfaces 7 of the cheeks 6a, 6b a channel 8 is formed having a funnel shaped opening 9 on one of its ends for facilitating the introduction of wires.

The crimping device is used in a way that the cheeks 6a, 6b are separated by moving the cheeks which are connected with a hinge 10. A sleeve 1 is put into the recess of the surface of the lower cheek 6a and the cheeks are closed again. Afterwards the plurality of wires 2 is fed through the channel 8 and the sleeve.

The sleeve is crimped by manually operating the lever 11 which rotates an extender wheel 12 by means of which a bolt 13 is moved into the recess of the upper cheek 6b thus crimping the sleeve with it tip.

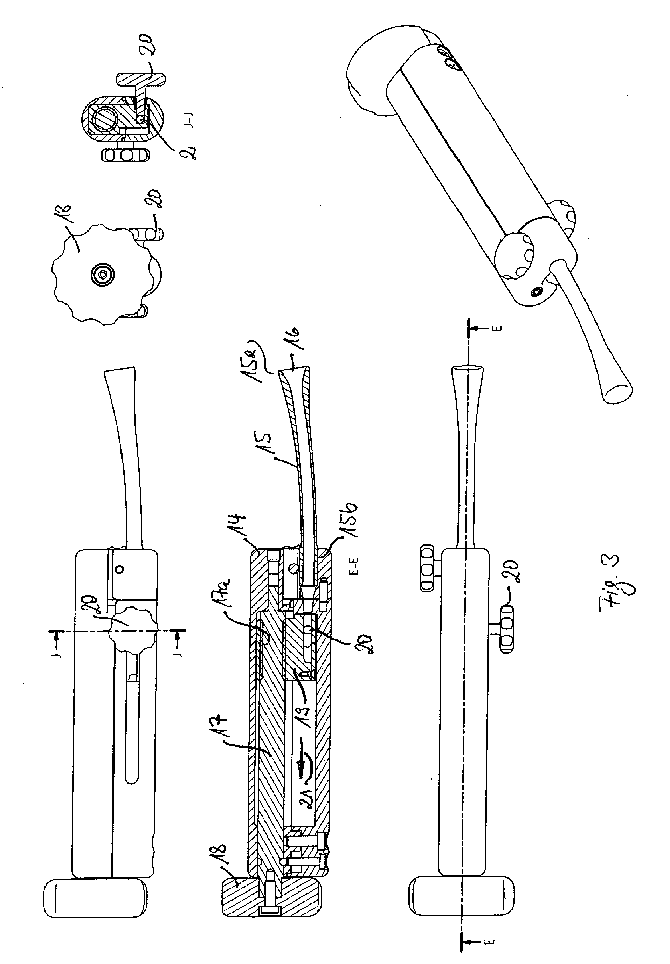

All the wires that are connected on one side to a stent and on the other side crimped together in the sleeve are now fed into the stent removal device shown in figure 3. This stent removal device comprises a support element 14 and a tube 15. One end 15a of the tube forms a funnel shaped opening 16. The other end 15b is attached to the support element 14, preferably by means of clamping.

The support element 14 furthermore comprises a drive mechanism having a rotatable axis 17 which is parallel to the end 15b of the tube or the wires therein and not shown in figure 3.

The axis is manually operated by a knob or handle 18 and has a threaded part 17a that is engaged by a sliding element 19. The wires being prepared with the sleeve may now be fed through the tube 15 into and through the sliding element 19. The wires may be clamped and fastened in the sliding element 19 by tightly screwing the screw 20. When operating the rotatable axis 17 with the knob 18 the sliding element is moved parallel to the end 15b of the tube 15 and the wires in the direction of arrow 21 thus pulling the wires into the tube together with the stent that is constricted due to the pulling force that is exerted to the stent via the wires evenly attached around the circumference of the stent at the proximal end.

As can be seen in figures 3 the tube 15 is slightly bent towards the axis of rotation 17 while the end 15b that is attached to the support element 14 is parallel to and offset from the axis 17.

The support element 14 is formed as a grip or handle and may accordingly manually used by a surgeon.

## Claims

1. Stent removal device comprising a tube (15) for feeding wires through the tube (15), the tube (15) having a distal end (15a) with a funnel shaped opening (16) for at least partially receiving a stent and a proximal end (15b), **characterized in that** the proximal end (15b) is connected to a supporting element (14, the supporting element (14) comprising a drive mechanism (17, 18, 19, 20) for fastening, particularly clamping and simultaneously pulling a plurality of wires fed through the tube (15).

2. Device according to claim 1, **characterized in that** the drive mechanism comprises a rotatable axis, particularly manually rotatable axis, the axis being supported by the supporting element and perpendicular to the direction of the extension of the plurality of wires in the proximal end of the tube and being capable to fasten the ends of the plurality of wires and to pull the plurality of wires through the tube by winding up their ends on the axis.

3. Device according to claim 1, **characterized in that** the drive mechanism (17, 18, 19, 20) comprises
a. a rotatable axis (17), particularly manually rotatable axis (17), the axis (17) being supported by the supporting element (14) and parallel to the direction of the extension of the plurality of wires in the proximal end (15b) of the tube (15) and
b. a sliding element (19), the sliding element (19) being engaged with a threaded part (17a) of the axis (17) and being slidable within the supporting element (14) in a direction parallel to the axis (17) by rotating the axis (17), the sliding element (19) furthermore comprising a clamping element (20) for fastening the respective end parts of the plurality of wires fed through the tube (15) and fed at least into the sliding element (19), preferably through the sliding element (19).

4. Device according to claim 2 or 3, **characterized in that** the rotatable axis (17) is connected to a manually operable knob (18), the knob (18) protruding from the supporting element (14).

5. Device according to anyone of the preceding claims, **characterized in that** the supporting element (14) forms a grip for manually gripping with a hand.

6. Device according to anyone of the preceding claims, **characterized in that** a clamping element (20) for fastening the respective end parts of the plurality of wires is formed of a screw (20) screwed into a threaded bore hole of the sliding element (19), the axis of the screw (20) crossing the axis of the wires fed into or through the sliding element (19).

7. Device according to anyone of the preceding claims, **characterized in that** the rotatable axis (17) and the middle axis of the proximal end (15b) of the tube (15) are parallel and offset.

8. Device according to anyone of the preceding claims, **characterized in that** the part of the tube (15) emerging from the supporting element (14) is bent in a plane, particularly in a plane comprising both axis according to claim 7.

9. Sleeve (1) comprising an axial through hole, particularly a bore and a funnel shaped opening (3) of the through hole on one of its ends for facilitating feeding a plurality of wires (2) through the sleeve (1), the sleeve (1) being crimpable with a crimping device for fastening the sleeve (1) on the plurality of wires (2), particularly for facilitating feeding the connected plurality of wires (2) through the tube (15) and or sliding element (19) of a device according to anyone of claims 1 to 8.

10. Sleeve according to claim 9, **characterized in that** the external diameter of the sleeve (1) is tapered, particularly in the middle of the axial extension.

11. Crimping device, particularly for crimping a sleeve (1) according to claims 9 or 10, **characterized in that** it comprises
a. an upper cheek (6b) and a lower cheek (6a), both cheeks (6a, 6b) being movable with respect to each other for opening a respective recess in each of the opposing surfaces of the respective cheeks (6a, 6b), particularly by means of a hinge (10) connecting both cheeks (6a, 6b), the recesses being adapted to receive a sleeve (1) according to claims 9 or 10,
b. in the closed state of the cheeks (6a, 6b) a channel (8) crossing the recesses and being collinear with the through hole of an inserted sleeve (1), particularly a part of the channel (8) forming a funnel (9) merging into the funnel of an inserted sleeve (1).
c. a bolt (13) in one of the cheeks (6a, 6b), particularly the upper cheek (6b), being movable in a direction perpendicular to the channel (8) and crossing at least one of the recesses and being adapted to crimp a sleeve (1) inserted in the recesses with its tip
d. a driving mechanism (11), particularly a manually operable lever (11), for driving the bolt (13).

12. Set comprising a device according to anyone of the claims 1 to 8, at least one sleeve (1) according to claim 9 and a crimping device according to anyone of claims 10 or 11.

13. Set according to claim 12 comprising a plurality (2) of at least 3 wires, particularly made of metal.
